# EUROPEAN PATENT SPECIFICATION

(11) **EP 3 831 309 B1**
(45) Date of publication and mention of the grant of the patent: **18.09.2024**
(21) Application number: 19889512.0
(22) Date of filing: 13.11.2019
(51) Int. Cl.: A61B 17/068, A61B 17/128

(54) **DUAL-MOTOR CLIP APPLYING FORCEPS**
ZWEIMOTORIGE CLIP-ANLEGEZANGE
PINCE À DOUBLE MOTEUR APPLIQUANT UNE AGRAFE

(30) Priority: 29.11.2018 CN 201811439560
(43) Date of publication of application: 09.06.2021
(73) Proprietor: Intocare Medical Technology (Suzhou) Co., Ltd., Suzhou, Jiangsu 215000 (CN)
(72) Inventor: ZHOU, Bingchao, Suzhou, Jiangsu 215000 (CN); ZHANG, Hui, Suzhou, Jiangsu 215000 (CN); DU, Yunfeng, Suzhou, Jiangsu 215000 (CN); LIU, Dianchen, Suzhou, Jiangsu 215000 (CN)
(74) Representative: Gulde & Partner
(86) International application number: PCT/CN2019/117885
(87) International publication number: WO 2020/108300

(56) References cited:
- EP-A2- 0 646 356
- CN-A- 1 665 449
- CN-A- 106 388 893
- CN-A- 107 405 149
- CN-U- 202 184 762
- US-A- 5 467 911
- US-A1- 2014 025 105
- US-A1- 2016 367 239
- US-A1- 2017 224 330

## Description

### TECHNICAL FIELD

The present disclosure relates to a dual-motor clip applicator, and belongs to the field of medical instruments.

### BACKGROUND

In order to fully expose a surgical visual field during a surgical operation, target tissue at an incision needs to be ligated to stop bleeding. Hemostasis technique has become one of main surgical techniques. Surgical operations on any part of human body almost without exception involve bleeding and stopping bleeding. Generally, a ligation clip applied by a clip applicator is used to clamp the target tissue so as to stop bleeding. A conventional clip applicator is usually single-firing and only applies one ligation clip at one time, and another ligation clip needs to be placed into the clip applicator after the one ligation clip clamps the target blood vessel. However, during the surgical operation, a plurality of ligation clips are required so as to stop bleeding; in this case, the conventional clip applicator has to enter and exit a puncture many times for example during an endoscopic surgical operation, which causes the time of the surgical operation to be too long, increases the bleeding amount of the patient, and adversely affects postoperative recovery of the patient. In addition, the conventional clip applicator completes the delivery of the ligation clip and the firing of the ligation clip only by one shaft, and has strong dependent and restrictive relationships between the delivery of the ligation clip and the firing of the ligation clip so that operation errors are easily caused.

US 5 467 911 A discloses a surgical device for applying surgical fasteners to body tissues in order to fasten the body tissues together. The device comprises an operation section having a housing having an inner space completely sealed from the outside, drive elements electrically driven and arranged in the inner space of the operation section, an insertion section including a housing which has a first end portion containing the fasteners and having an opening through which the fasteners are to be applied, and a second end portion removably coupled to the housing of the operation section, fastener-applying elements located in the housing of the insertion section, for applying the fasteners from the opening to body tissues, and force-transmitting elements for transmitting drive force from the drive elements to the fastener-applying elements.

US 2014/025105 A1 discloses a surgical device including a first jaw and a second jaw is presented. The surgical device also includes a biasing element that biases the distal end of the first jaw towards the distal end of the second jaw. The device also includes a first driver disposed in the second jaw and coupled to the first jaw. The first driver is configured to cause separation of the first jaw and the second jaw. The device further includes at least one of cutting element and a stapling element disposed within the second jaw, preferably a blade rotatably mounted on a wedge. A second driver is configured to move the cutting element and/or the stapling element proximally from a distal end toward the proximal end of the second jaw to at least one of cut and staple a section of tissue disposed between the first and second jaws.

### SUMMARY

The present disclosure aims to provide a dual-motor clip applicator to solve the problems that the conventional clip applicator needs to complete the firing of the ligation clip and the delivery of the ligation clip by one shaft and thus has strong dependent and restrictive relationships between the firing of the ligation clip and the delivery of the ligation clip to easily cause operational errors and the like; the dual-motor clip applicator provided by the embodiments of the present disclosure uses two motors, such that the operation is simpler, the freedom degree of the operation is higher, and the error rate is reduced.

In order to achieve the above objects, the technical solutions of the present disclosure are provided as follows. A dual-motor clip applicator comprises a working head, and a first motor, a second motor, a first driving assembly, a second driving assembly and a clip cartridge assembly that are provided in the working head. The clip cartridge assembly comprises a clip cartridge configured to accommodate a ligation clip and a clip jaw provided on a side of the clip cartridge; the working head further comprises a firing rod and a clip-pushing rod, the firing rod is connected to the clip jaw to drive the clip jaw to open or close for firing the ligation clip, the first driving assembly is respectively connected to the firing rod and the first motor, the second driving assembly is respectively connected to the clip-pushing rod and the second motor, and the clip-pushing rod is configured to push the ligation clip in the clip cartridge to enter into the clip jaw.

The above description is only an outline of the technical solutions of the embodiments of the present disclosure. In order to better understand the technical solutions of the embodiments of the present disclosure so that they can be implemented according to the contents of the specification, the following detailed description will be made with the exemplary embodiments of the present disclosure in conjunction with the accompanying drawings.

### BRIEF DESCRIPTION OF THE DRAWINGS

FIG. 1 is an external structural schematic diagram of a dual-motor clip applicator according to embodiments of the present disclosure.
FIG. 2 is an internal structural schematic diagram of the dual-motor clip applicator according to the embodiments of the present disclosure.
FIG. 3 is a structural schematic diagram of motors and drive assemblies of the dual-motor clip applicator according to the embodiments of the present disclosure.
FIG. 4 is a structural schematic diagram of a clip cartridge and a clip jaw of the dual-motor clip applicator according to the embodiments of the present disclosure.
FIG. 5 is a structural schematic diagram of a support sleeve of the dual-motor clip applicator according to the embodiments of the present disclosure.

### DETAILED DESCRIPTION

The exemplary specific modes of the present disclosure will be described in further detail below in conjunction with the drawings and embodiments. The following embodiments are intended to illustrate the present disclosure, but not to limit the scope of the present disclosure.

It should be noted that the terms such as "upper", "lower", "left", "right", "inner", "outer" and the like in the present disclosure are only intended to describe the embodiments of the present disclosure by referring to the drawings, and are not used as limiting terms.

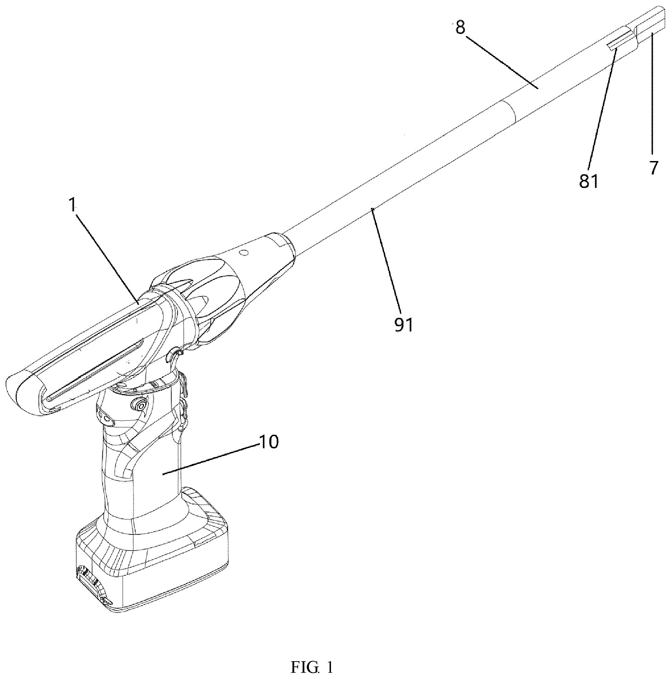

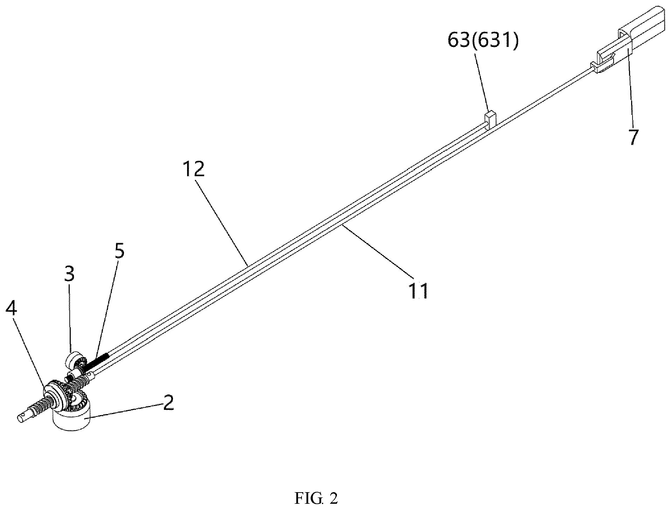

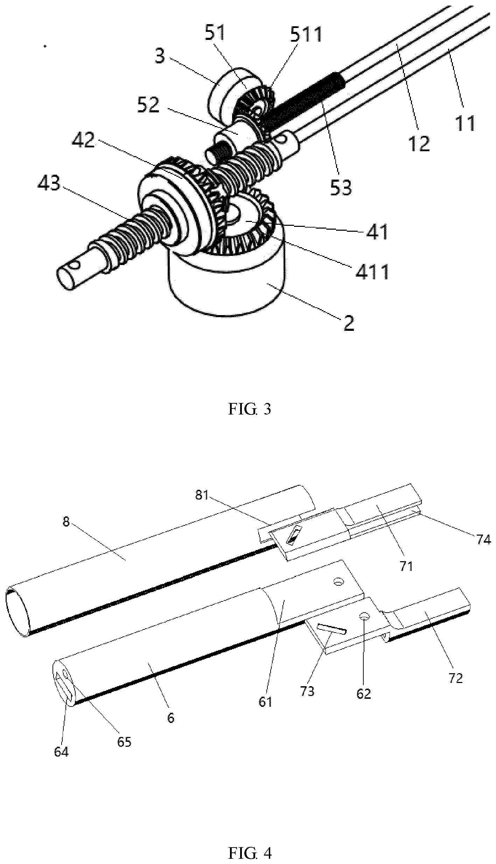

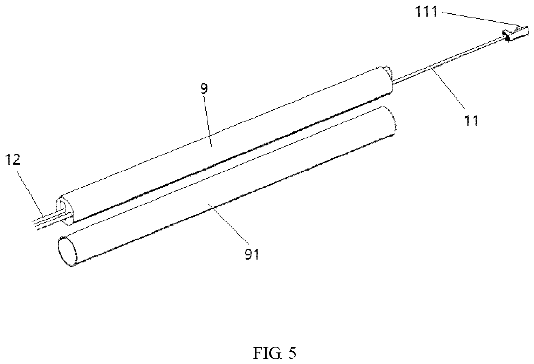

Referring to FIG. 1 to FIG. 5, a dual-motor clip applicator is provided by embodiments of the present disclosure, the dual-motor clip applicator includes a working head 1, and a first motor 2, a second motor 3, a first driving assembly 4, a second driving assembly 5 and a clip cartridge assembly that are provided in the working head 1; the clip cartridge assembly includes a clip cartridge 6 configured to accommodate a ligation clip and a clip jaw 7 provided on a side of the clip cartridge 6; a firing rod 11 and a clip-pushing rod 12 are further provided in the working head 1, the firing rod 11 is connected to the clip jaw 7 to drive the clip jaw 7 to open or close for firing the ligation clip, the first driving assembly 4 is respectively connected to the firing rod 11 and the first motor 2, the second driving assembly 5 is respectively connected to the clip-pushing rod 12 and the second motor 3, and the clip-pushing rod 12 is configured to push the ligation clip in the clip cartridge 6 to enter into the clip jaw 7.

In the embodiments of the present disclosure, the first driving assembly 4 includes a first driving gear 41, a first nut 42 and a first threaded rod 43, the first driving gear 41 is provided on the first motor 2, gears 411 engaging with each other are respectively provided on the first driving gear 41 and the first nut 42, the first threaded rod 43 is connected to the first nut 42 by thread connection manner, and the first threaded rod 43 is connected to the firing rod 11. The second driving assembly 5 includes a second driving gear 51, a second nut 52 and a second threaded rod 53, the second driving gear 51 is provided on the second motor 3, gears 511 engaging with each other are respectively provided on the second driving gear 51 and the second nut 52, the second threaded rod 53 is connected to the second nut 52 by thread connection manner, and the second threaded rod 53 is connected to the clip-pushing rod 12.

In the embodiments of the present disclosure, an end of the clip cartridge 6 is provided with a mounting part 61, and the clip jaw 7 is mounted to the mounting part 61. For example, a mounting hole 62 is respectively provided on the clip jaw 7 and the mounting part 61; and a mounting pin (not shown) is inserted into the mounting holes 62 so that the clip jaw 7 is mounted to the mounting part 61. The clip jaw 7 includes a first clip jaw sheet 71 and a second clip jaw sheet 72, a connecting groove 73 is respectively provided on the first clip jaw sheet 71 and the second clip jaw sheet 72, and the connecting groove 73 provided on the first clip jaw sheet 71 and the connecting groove 73 provided on the second clip jaw sheet 72 cross with each other. A positioning groove 74 is provided on the first clip jaw sheet 71 and the second clip jaw sheet 72. After entering the clip jaw 7, the ligation clip is accommodated in the positioning grooves 74. A connecting pin 111 is provided on an end, close to the clip jaw 7, of the firing rod 11, and the connecting pin 111 is fixedly provided in the connecting grooves 73, such that the firing rod 11 drives the clip jaw 7 to open or close for firing the ligation clip. In the embodiments, an X-shaped arrangement is used. The clip jaw 7 is mounted on the mounting part 61; if the firing rod 11 pushes the clip jaw 7 outwards, the connecting pin 111 provides an outward push force for the first clip jaw sheet 71 and the second clip jaw sheet 72, such that the clip jaw 7 opens; and if the firing rod 11 pulls the clip jaw 7 inwards, the connecting pin 111 provides an inward pull force for the first clip jaw sheet 71 and the second clip jaw sheet 72, such that the clip jaw 7 closes and fires.

For example, a clip delivery assembly 63 configured for pushing the ligation clip to move in the clip cartridge 6 is provided in the clip cartridge 6. The clip delivery assembly 63 includes a clip pushing block 631. The clip pushing block 631 abuts against and pushes the ligation clip, and the clip-pushing rod 12 is connected to the clip pushing block 631. In the embodiments, the clip delivery assembly 63 for example further includes a spring. The spring is connected with the clip pushing block 631 and plays a reset role. A first channel 64 and a second channel 65 are provided on the clip cartridge 6. The clip-pushing rod 12 and the clip delivery assembly 63 are provided in the first channel 64, and the firing rod 11 is provided in the second channel 65. The second driving assembly 5 controls a position of the clip pushing block 631 in the clip cartridge 6 by driving the clip-pushing rod 12 to move, so as to drive the ligation clip to move. With the above arrangement, the electric and continuous firing of the ligation clips in the clip cartridge 6 is implemented; and theoretically, there are no limits made on the total number of the ligation clips in the clip cartridge 6.

In the embodiments of the present disclosure, the clip cartridge assembly further includes an outer sleeve 8, the outer sleeve 8 is provided in the working head 1 and is sleeved on the clip cartridge 6, and a cutout 81 for the opening or firing of the clip jaw 7 is provided at an end, close to the clip jaw 7, of the outer sleeve 8. The working head 1 further includes a support sleeve 9 that is connected to the outer sleeve 8 and is also provided with the first channel 64 and the second channel 65, and the firing rod 11 and the clip-pushing rod 12 are respectively provided in the first channel 64 and the second channel 65 of each of the support sleeve 9 and the clip cartridge 6. With such a design, the support sleeve 9 is used repeatedly without replacement, but the clip cartridges 6 of different models and specifications are mounted for surgical operation. In the embodiments, a protective sleeve 91 is sleeved on the support sleeve 9, and the protective sleeve 91 is connected to the outer sleeve 8.

The dual-motor continuous-firing clip applicator provided by the embodiments of the present disclosure further includes a handle 10, a control circuit (not shown in the figures) is provided in the handle 10, the control circuit is electrically connected to the first motor 2 and the second motor 3, and the control circuit comprises a motor control module, a support sleeve identification module, a clip delivery and clip firing control module, a battery detection and control module, a working status indicator module and a data record storage module that are controlled by single-chip processor.

In the control circuit:
The motor control module is configured for controlling the switching-on and switching-off of a drive motor; a closing/opening button (not shown) mounted at a lower portion of the handle 10 is isolated from an inner part of the main body of the clip applicator; and the motor control is realized by a magnet inside the button being close to or away from a Hall switch sealed and packaged in the handle.

The support sleeve identification module transmits the data about the type of the support sleeve by a terminal (not shown) provided on the support sleeve 8, and then the control circuit starts a corresponding working program based on the data.

The clip delivery and clip firing control module, together with a signal identification module (not shown), a photoelectric limit switch position control module, and a safety module that are mounted in the support sleeve 8, realizes the delivery of the ligation clip and the firing of the ligation clip.

The battery detection and control module provides a battery power detection function, and monitors the battery power before and during the surgical operation to ensure that the battery power used during the surgical operation meets the needs of the surgical operation or the battery is replaced in time.

The working status indicator module provides a status of the battery power, a support sleeve connection status, a status of completing the delivery of the ligation clip, and a status of completing the firing of the ligation chip by a display.

The dual-motor clip applicator provided by the embodiments of the present disclosure operates as follows. Initially, a plurality of ligation clips are provided in the clip cartridge 6, and the clip pushing block 631 connected to the clip-pushing rod 12 is provided at a side of the ligation clips away from the clip jaw 7; during the process of delivering the ligation clip into the clip jaw, by pressing the button on the handle 10, the firing rod 11 drives the clip jaw 7 to open, and at this time, the clip-pushing rod 12 is pushed forwards, thereby driving the clip pushing block 631 to push the ligation clip to enter the positioning groove 74 of the clip jaw 7; after the ligation clip reaches a position of a target vessel or tissue, by pressing the button on the handle 10, the firing rod 11 drives the clip jaw 7 to pull back and close such that the target vessel or tissue is ligated, thereby completing the firing. Then, the above procedures are repeated to complete the firing of the next ligation clip.

Compared with the prior art, the beneficial effects of the embodiments of the present disclosure at least are as follows. The dual-motor clip applicator provided by the embodiments of the present disclosure uses the first motor and the second motor to respectively control the firing of the clip jaw and the clip delivery of the clip cartridge, such that the operation is simpler, the freedom degree of the operation is higher, the error rate is reduced, and the problems that the conventional clip applicator needs to complete the firing of the ligation clip and the delivery of the ligation clip by one shaft and thus has strong dependent and restrictive relationships between the firing of the ligation clip and the delivery of the ligation clip to easily cause operational errors and the like are solved.

Furthermore, according to the dual-motor clip applicator, the working head is separated into the support sleeve and the clip cartridge assembly that are connected to each other, the clip delivery assembly is provided in the clip cartridge, and the firing rod and the clip-pushing rod are provided in the support sleeve and the clip cartridge; and thus, the support sleeve is capable of being reused, so that clip cartridge assemblies of different specifications are mounted to meet the surgical requirements, thereby greatly reducing the cost and reducing the number of surgical instruments.

In addition, the clip applicator of the embodiments of the present disclosure works automatically, is electrically driven and does not rely on the experience and status of a doctor; therefore, the stability of a ligation effect is good, and the doctor does not need to fire manually, which saves the doctor's physical strength, time and effort.

The technical features of the foregoing embodiments may be combined arbitrarily. In order to make the description concise, all possible combinations of the various technical features in the foregoing embodiments are not described. However, as long as there is no conflict in the combination of these technical features, all combinations should be considered as within the scope of the disclosure.

The foregoing embodiments merely are some of the embodiments of the present disclosure and the descriptions of the foregoing embodiments are specific and detailed, but the foregoing embodiments should not be understood as limiting of the scope of the disclosure. It should be pointed out that a person of ordinary skill in the art may make modifications and improvements without departing from the scope of the appended claims, and all of these modifications and improvements belong to the protection scope of the present disclosure as defined by the appended claims. Therefore, the protection scope of the present disclosure should be defined by the appended claims.

## Claims

1. A dual-motor clip applicator, comprising a working head (1), and a handle (10), wherein the working head (1) comprises: a first motor (2), a second motor (3), a first driving assembly (4), a second driving assembly (5), and a clip cartridge assembly that are provided in the working head (1),
the clip cartridge assembly comprises a clip cartridge (6) configured to accommodate a ligation clip and a clip jaw (7) provided on a side of the clip cartridge (6);
the working head (1) further comprises a firing rod (11) and a clip-pushing rod (12), the firing rod (11) is connected to the clip jaw (7) to drive the clip jaw (7) to open or close for firing the ligation clip, the first driving assembly (4) is respectively connected to the firing rod (11) and the first motor (2), the second driving assembly (5) is respectively connected to the clip-pushing rod (12) and the second motor (3), and the clip-pushing rod (12) is configured to push the ligation clip in the clip cartridge (6) to enter into the clip jaw (7).

2. The dual-motor clip applicator according to claim 1, wherein the first driving assembly (4) comprises a first driving gear (41), a first nut (42) and a first threaded rod (43), the first driving gear (41) is provided on the first motor (2), gears engaging with each other are respectively provided on the first driving gear (41) and the first nut (42), the first threaded rod (43) is connected to the first nut (42) by thread connection manner, and the first threaded rod (43) is connected to the firing rod (11).

3. The dual-motor clip applicator according to claim 1, wherein the second driving assembly (5) comprises a second driving gear (51), a second nut (52) and a second threaded rod (53), the second driving gear (51) is provided on the second motor (3), gears engaging with each other are respectively provided on the second driving gear (51) and the second nut (52), the second threaded rod (53) is connected to the second nut (52) by thread connection manner, and the second threaded rod (53) is connected to the clip-pushing rod (12).

4. The dual-motor clip applicator according to claim 1, wherein an end of the clip cartridge (6) is provided with a mounting part (61), the clip jaw (7) is mounted to the mounting part (61).

5. The dual-motor clip applicator according to claim 4, wherein the clip jaw (7) comprises a first clip jaw sheet (71) and a second clip jaw sheet (72), a connecting groove (73) is respectively provided on the first clip jaw sheet (71) and the second clip jaw sheet (72), the connecting groove (73) provided on the first clip jaw sheet (71) and the connecting groove (73) provided on the second clip jaw sheet (72) cross with each other, a connecting pin (111) is provided on an end, close to the clip jaw (7), of the firing rod (11), and the connecting pin (111) is provided in the connecting grooves (73) of the first clip jaw sheet (71) and the second clip jaw sheet (72).

6. The dual-motor clip applicator according to claim 1, wherein a clip delivery assembly (63) configured for pushing the ligation clip to move in the clip cartridge (6) is provided in the clip cartridge (6), a first channel (64) and a second channel (65) are provided on the clip cartridge (6), the clip-pushing rod (12) and the clip delivery assembly (63) are provided in the first channel (64), and the firing rod (11) is provided in the second channel (65).

7. The dual-motor clip applicator according to claim 6, wherein the clip delivery assembly (63) comprises a clip pushing block (631), the clip pushing block (631) abuts against and pushes the ligation clip, the clip-pushing rod (12) is connected to the clip pushing block (631), and the second driving assembly (5) controls a position of the clip pushing block (631) in the clip cartridge (6) by driving the clip-pushing rod (12) to move, so as to drive the ligation clip to move.

8. The dual-motor clip applicator according to claim 6, wherein an outer sleeve (8) is sleeved on the clip cartridge (6), and a cutout (81) for opening or firing of the clip jaw (7) is provided at an end, close to the clip jaw (7), of the outer sleeve (8).

9. The dual-motor clip applicator according to claim 8, wherein the working head (1) further comprises a support sleeve (9), the support sleeve (9) is connected to the outer sleeve (8), the first channel (64) and the second channel (65) are further provided in the support sleeve (9), and the firing rod (11) and the clip-pushing rod (12) are respectively provided in the first channel (64) and the second channel (65) of each of the support sleeve (9) and the clip cartridge (6).

10. The dual-motor clip applicator according to claim 1, wherein a control circuit is provided in the handle (10), and the control circuit is electrically connected to the first motor (2) and the second motor (3).

11. The dual-motor clip applicator according to claim 2, wherein the second driving assembly (5) comprises a second driving gear (51), a second nut (52) and a second threaded rod (53), the second driving gear (51) is provided on the second motor (3), gears engaging with each other are respectively provided on the second driving gear (51) and the second nut (52), the second threaded rod (53) is connected to the second nut (52) by thread connection manner, and the second threaded rod (53) is connected to the clip-pushing rod (12).

12. The dual-motor clip applicator according to claim 4, wherein a mounting hole (62) is respectively on the clip jaw (7) and the mounting part (61), and a mounting pin is inserted into the mounting holes (62) of the clip jaw (7) and the mounting part (61) so that the clip jaw (7) is mounted to the mounting part (61).

13. The dual-motor clip applicator according to claim 5, wherein a positioning groove (74) is provided on the first clip jaw sheet (71) and the second clip jaw sheet (72); and after entering into the clip jaw (7), the ligation clip is accommodated in the positioning grooves (74).

14. The dual-motor clip applicator according to claim 7, wherein the clip delivery assembly (63) further includes a spring, and the spring is connected with the clip pushing block (631) and plays a reset role.

15. The dual-motor clip applicator according to claim 9, wherein a protective sleeve (91) is sleeved on the support sleeve (9), and the protective sleeve (91) is connected to the outer sleeve (8).

## Patentansprüche

1. Zweimotoriger Clip-Anleger, umfassend einen Arbeitskopf (1) und einen Griff (10), wobei der Arbeitskopf (1) Folgendes umfasst: einen ersten Motor (2), einen zweiten Motor (3), eine erste Antriebsbaugruppe (4), eine zweite Antriebsbaugruppe (5) und eine Clip-Kartuschen-Baugruppe, die in dem Arbeitskopf (1) bereitgestellt sind,
wobei die Clip-Kartuschen-Baugruppe eine Clip-Kartusche (6) umfasst, die dazu ausgestaltet ist, einen Abbinde-Clip und eine Clip-Backe (7) unterzubringen, die auf einer Seite der Clip-Kartusche (6) bereitgestellt sind;
wobei der Arbeitskopf (1) ferner einen Betätigungsstab (11) und einen Clip-Schiebestab (12) umfasst, wobei der Betätigungsstab (11) mit der Clip-Backe (7) verbunden ist, um die Clip-Backe (7) dazu zu drängen, sich zu öffnen oder zum Betätigen des Abbinde-Clips zu schließen, die erste Antriebsbaugruppe (4) jeweils mit dem Betätigungsstab (11) und dem ersten Motor (2) verbunden ist, die zweite Antriebsbaugruppe (5) jeweils mit dem Clip-Schiebestab (12) und dem zweiten Motor (3) verbunden ist und der Clip-Schiebestab (12) dazu ausgestaltet ist, den Abbinde-Clip in der Clip-Kartusche (6) zum Eintritt in die Clip-Backe (7) zu schieben.

2. Zweimotoriger Clip-Anleger nach Anspruch 1, wobei die erste Antriebsbaugruppe (4) ein erstes Triebwerk (41), eine erste Mutter (42) und einen ersten Gewindestab (43) umfasst, wobei das erste Triebwerk (41) an dem ersten Motor (2) bereitgestellt ist, Zahnräder, die einander in Eingriff nehmen, jeweils an dem ersten Triebwerk (41) und der ersten Mutter (42) bereitgestellt sind, der erste Gewindestab (43) mit der ersten Mutter (42) durch Gewindeverbindung verbunden ist und der erste Gewindestab (43) mit dem Betätigungsstab (11) verbunden ist.

3. Zweimotoriger Clip-Anleger nach Anspruch 1, wobei die zweite Antriebsbaugruppe (5) ein zweites Triebwerk (51), eine zweite Mutter (52) und einen zweiten Gewindestab (53) umfasst, wobei das zweite Triebwerk (51) an dem zweiten Motor (3) bereitgestellt ist, Zahnräder, die einander in Eingriff nehmen, jeweils an dem zweiten Triebwerk (51) und der zweiten Mutter (52) bereitgestellt sind, der zweite Gewindestab (53) mit der zweiten Mutter (52) durch Gewindeverbindung verbunden ist und der zweite Gewindestab (53) mit dem Clip-Schiebestab (12) verbunden ist.

4. Zweimotoriger Clip-Anleger nach Anspruch 1, wobei ein Ende der Clip-Kartusche (6) mit einem Befestigungsteil (61) versehen ist, wobei die Clip-Backe (7) an dem Befestigungsteil (61) befestigt ist.

5. Zweimotoriger Clip-Anleger nach Anspruch 4, wobei die Clip-Backe (7) ein erstes Clip-Backenblech (71) und ein zweites Clip-Backenblech (72) umfasst, eine Verbindungsnut (73) jeweils an dem ersten Clip-Backenblech (71) und dem zweiten Clip-Backenblech (72) bereitgestellt ist, die an dem ersten Clip-Backenblech (71) bereitgestellte Verbindungsnut (73) und die an dem zweiten Clip-Backenblech (72) bereitgestellte Verbindungsnut (73) einander kreuzen, ein Verbindungsstift (111) an einem Ende, nahe der Clip-Backe (7), des Betätigungsstabs (11) bereitgestellt ist und der Verbindungsstift (111) in den Verbindungsnuten (73) des ersten Clip-Backenblechs (71) und des zweiten Clip-Backenblechs (72) bereitgestellt ist.

6. Zweimotoriger Clip-Anleger nach Anspruch 1, wobei eine Clip-Abgabebaugruppe (63), die zum Schieben des Abbinde-Clips zur Bewegung in der Clip-Kartusche (6) ausgestaltet ist, in der Clip-Kartusche (6) bereitgestellt ist, ein erster Kanal (64) und ein zweiter Kanal (65) an der Clip-Kartusche (6) bereitgestellt sind, wobei der Clip-Schiebestab (12) und die Clip-Abgabebaugruppe (63) in dem ersten Kanal (64) bereitgestellt sind und der Betätigungsstab (11) in dem zweiten Kanal (65) bereitgestellt ist.

7. Zweimotoriger Clip-Anleger nach Anspruch 6, wobei die Clip-Abgabebaugruppe (63) einen Clip-Schiebeblock (631) umfasst, wobei der Clip-Schiebeblock (631) an den Abbinde-Clip anstößt und ihn schiebt, der Clip-Schiebestab (12) mit dem Clip-Schiebeblock (631) verbunden ist und die zweite Antriebsbaugruppe (5) eine Position des Clip-Schiebeblocks (631) in der Clip-Kartusche (6) steuert, indem sie den Clip-Schiebestab (12) antreibt, sich zu bewegen, um den Abbinde-Clip anzutreiben, sich zu bewegen.

8. Zweimotoriger Clip-Anleger nach Anspruch 6, wobei ein Außenmantel (8) die Clip-Kartusche (6) ummantelt und ein Ausschnitt (81) zum Öffnen oder Betätigen der Clip-Backe (7) an einem Ende, nahe der Clip-Backe (7), des Außenmantels (8) bereitgestellt ist.

9. Zweimotoriger Clip-Anleger nach Anspruch 8, wobei der Arbeitskopf (1) ferner einen Stützmantel (9) umfasst, wobei der Stützmantel (9) mit dem Außenmantel (8) verbunden ist, der erste Kanal (64) und der zweite Kanal (65) ferner in dem Stützmantel (9) bereitgestellt sind und der Betätigungsstab (11) und der Clip-Schiebestab (12) jeweils in dem ersten Kanal (64) und dem zweiten Kanal (65) von jedem von dem Stützmantel (9) und der Clip-Kartusche (6) bereitgestellt sind.

10. Zweimotoriger Clip-Anleger nach Anspruch 1, wobei ein Steuerkreis in dem Griff (10) bereitgestellt ist und der Steuerkreis elektrisch mit dem ersten Motor (2) und dem zweiten Motor (3) verbunden ist.

11. Zweimotoriger Clip-Anleger nach Anspruch 2, wobei die zweite Antriebsbaugruppe (5) ein zweites Triebwerk (51), eine zweite Mutter (52) und einen zweiten Gewindestab (53) umfasst, wobei das zweite Triebwerk (51) an dem zweiten Motor (3) bereitgestellt ist, Zahnräder, die einander in Eingriff nehmen, jeweils an dem zweiten Triebwerk (51) und der zweiten Mutter (52) bereitgestellt sind, der zweite Gewindestab (53) mit der zweiten Mutter (52) durch Gewindeverbindung verbunden ist und der zweite Gewindestab (53) mit dem Clip-Schiebestab (12) verbunden ist.

12. Zweimotoriger Clip-Anleger nach Anspruch 4, wobei sich ein Befestigungsloch (62) jeweils an der Clip-Backe (7) und dem Befestigungsteil (61) befindet und ein Befestigungsstift derart in die Befestigungslöcher (62) der Clip-Backe (7) und des Befestigungsteils (61) eingesetzt ist, dass die Clip-Backe (7) an dem Befestigungsteil (61) befestigt ist.

13. Zweimotoriger Clip-Anleger nach Anspruch 5, wobei eine Positionierungsnut (74) an dem ersten Clip-Backenblech (71) und dem zweiten Clip-Backenblech (72) bereitgestellt ist; und nach dem Eintritt in die Clip-Backe (7) der Abbinde-Clip in den Positionierungsnuten (74) untergebracht ist.

14. Zweimotoriger Clip-Anleger nach Anspruch 7, wobei die Clip-Abgabebaugruppe (63) ferner eine Feder aufweist und die Feder mit dem Clip-Schiebeblock (631) verbunden ist und eine Rücksetzrolle spielt.

15. Zweimotoriger Clip-Anleger nach Anspruch 9, wobei ein Schutzmantel (91) den Stützmantel (9) ummantelt und der Schutzmantel (91) mit dem Außenmantel (8) verbunden ist.

## Revendications

1. Applicateur d'agrafes à double moteur, comprenant une tête de travail (1) et une poignée (10), dans lequel la tête de travail (1) comprend : un premier moteur (2), un second moteur (3), un premier ensemble d'entraînement (4), un second ensemble d'entraînement (5) et un ensemble de cartouche d'agrafes prévus dans la tête de travail (1),
l'ensemble de cartouche d'agrafes comprend une cartouche d'agrafes (6) configurée pour recevoir une agrafe de ligature et une mâchoire de serrage (7) prévues sur un côté de la cartouche d'agrafes (6) ;
la tête de travail (1) comprend en outre une tige de déclenchement (11) et une tige de poussée de l'agrafe (12), la tige de déclenchement (11) est reliée à la mâchoire de serrage (7) pour entraîner l'ouverture ou la fermeture de la mâchoire de serrage (7) afin de déclencher l'agrafe de ligature, le premier ensemble d'entraînement (4) est respectivement relié à la tige de déclenchement (11) et au premier moteur (2), le second ensemble d'entraînement (5) est respectivement relié à la tige de poussée de l'agrafe (12) et au second moteur (3), et la tige de poussée de l'agrafe (12) est configurée pour pousser l'agrafe de ligature dans la cartouche d'agrafes (6) afin qu'elle pénètre dans la mâchoire de serrage (7).

2. Applicateur d'agrafes à double moteur selon la revendication 1, dans lequel le premier ensemble d'entraînement (4) comprend un premier engrenage d'entraînement (41), un premier écrou (42) et une première tige filetée (43), le premier engrenage d'entraînement (41) est prévu sur le premier moteur (2), des engrenages s'engageant l'un dans l'autre sont respectivement prévus sur le premier engrenage d'entraînement (41) et le premier écrou (42), la première tige filetée (43) est reliée au premier écrou (42) par un raccord fileté, et la première tige filetée (43) est reliée à la tige de déclenchement (11).

3. Applicateur d'agrafes à double moteur selon la revendication 1, dans lequel le second ensemble d'entraînement (5) comprend un second engrenage d'entraînement (51), un second écrou (52) et une seconde tige filetée (53), le second engrenage d'entraînement (51) est prévu sur le second moteur (3), des engrenages s'engageant l'un dans l'autre sont respectivement prévus sur le second engrenage d'entraînement (51) et le second écrou (52), la seconde tige filetée (53) est reliée au second écrou (52) par un raccord fileté, et la seconde tige filetée (53) est reliée à la tige de poussée de l'agrafe (12).

4. Applicateur d'agrafes à double moteur selon la revendication 1, dans lequel une extrémité de la cartouche d'agrafes (6) est pourvue d'une pièce de montage (61), la mâchoire de serrage (7) étant montée sur la pièce de montage (61).

5. Applicateur d'agrafes à double moteur selon la revendication 4, dans lequel la mâchoire de serrage (7) comprend une première plaque de serrage de la mâchoire (71) et une seconde plaque de serrage de la mâchoire (72), une rainure de raccordement (73) est respectivement prévue sur la première plaque de serrage de la mâchoire (71) et la seconde plaque de serrage de la mâchoire (72), la rainure de raccordement (73) de la première plaque de serrage de la mâchoire (71) et la rainure de raccordement (73) de la seconde plaque de serrage de la mâchoire (72) se croisent, une broche de raccordement (111) est prévue à une extrémité de la tige de déclenchement (11), à proximité de la mâchoire de serrage (7), et la broche de raccordement (111) est prévue dans les rainures de raccordement (73) de la première plaque de serrage de la mâchoire (71) et de la seconde plaque de serrage de la mâchoire (72).

6. Applicateur d'agrafes à double moteur selon la revendication 1, dans lequel un ensemble de distribution d'agrafes (63) configuré pour pousser l'agrafe de ligature à se déplacer dans la cartouche d'agrafes (6) est prévu dans la cartouche d'agrafes (6), un premier canal (64) et un second canal (65) sont prévus sur la cartouche d'agrafes (6), la tige de poussée de l'agrafe (12) et l'ensemble de distribution d'agrafes (63) sont prévus dans le premier canal (64), et la tige de déclenchement (11) est prévue dans le second canal (65).

7. Applicateur d'agrafes à double moteur selon la revendication 6, dans lequel l'ensemble de distribution d'agrafes (63) comprend un bloc de poussée de l'agrafe (631), le bloc de poussée de l'agrafe (631) vient en butée contre l'agrafe de ligature et la pousse, la tige de poussée de l'agrafe (12) est reliée au bloc de poussée de l'agrafe (631), et le second ensemble d'entraînement (5) commande une position du bloc de poussée de l'agrafe (631) dans la cartouche d'agrafes (6) en entraînant le déplacement de la tige de poussée de l'agrafe (12), de manière à entraîner le déplacement de l'agrafe de ligature.

8. Applicateur d'agrafes à double moteur selon la revendication 6, dans lequel un manchon extérieur (8) est monté sur la cartouche d'agrafes (6), et une découpe (81) pour l'ouverture ou le déclenchement de la mâchoire de serrage (7) est prévue à une extrémité, proche de la mâchoire de serrage (7), du manchon extérieur (8).

9. Applicateur d'agrafes à double moteur selon la revendication 8, dans lequel la tête de travail (1) comprend en outre un manchon de support (9), le manchon de support (9) est relié au manchon extérieur (8), le premier canal (64) et le second canal (65) sont en outre prévus dans le manchon de support (9), et la tige de déclenchement (11) et la tige de poussée de l'agrafe (12) sont respectivement prévues dans le premier canal (64) et le second canal (65) de chacun des manchon de support (9) et cartouche d'agrafes (6).

10. Applicateur d'agrafes à double moteur selon la revendication 1, dans lequel un circuit de commande est prévu dans la poignée (10), et le circuit de commande est relié de manière électrique au premier moteur (2) et au second moteur (3).

11. Applicateur d'agrafes à double moteur selon la revendication 2, dans lequel le second ensemble d'entraînement (5) comprend un second engrenage d'entraînement (51), un second écrou (52) et une seconde tige filetée (53), le second engrenage d'entraînement (51) est prévu sur le second moteur (3), des engrenages s'engageant l'un dans l'autre sont respectivement prévus sur le second engrenage d'entraînement (51) et le second écrou (52), la seconde tige filetée (53) est reliée au second écrou (52) par un raccord fileté, et la seconde tige filetée (53) est reliée à la tige de poussée de l'agrafe (12).

12. Applicateur d'agrafes à double moteur selon la revendication 4, dans lequel un trou de montage (62) se trouve respectivement sur la mâchoire de serrage (7) et la pièce de montage (61), et une broche de montage est insérée dans les trous de montage (62) de la mâchoire de serrage (7) et de la pièce de montage (61) de manière à ce que la mâchoire de serrage (7) soit montée sur la pièce de montage (61).

13. Applicateur d'agrafes à double moteur selon la revendication 5, dans lequel une rainure de positionnement (74) est prévue sur la première plaque de serrage de la mâchoire (71) et sur la seconde plaque de serrage de la mâchoire (72) ; et après avoir été introduit dans la mâchoire de serrage (7), l'agrafe de ligature est logée dans les rainures de positionnement (74).

14. Applicateur d'agrafes à double moteur selon la revendication 7, dans lequel l'ensemble de distribution d'agrafes (63) comprend en outre un ressort, et le ressort est relié au bloc de poussée de l'agrafe (631) et joue un rôle de remise en place.

15. Applicateur d'agrafes à double moteur selon la revendication 9, dans lequel un manchon de protection (91) est monté sur le manchon de support (9), et le manchon de protection (91) est relié au manchon extérieur (8).
